# EUROPÄISCHE PATENTSCHRIFT

(11) **EP 1 928 867 B1**
(45) Veröffentlichungstag und Bekanntmachung des Hinweises auf die Patenterteilung: **22.05.2013**
(21) Anmeldenummer: 06777189.9
(22) Anmeldetag: 14.09.2006
(51) Int. Cl.: C07D 413/12, A61K 31/42, A61P 7/00

(54) **2-AMINOETHOXYESSIGSÄURE-DERIVATE UND IHRE VERWENDUNG ZUR BEHANDLUNG THROMBOEMBOLISCHER ERKRANKUNGEN**
2-AMINOETHOXYACETIC ACID DERIVATIVES AND THEIR USE IN TREATING THROMBOEMBOLIC DISORDERS
DÉRIVÉS D'ACIDE 2-AMINOÉTHOXYACÉTIQUE ET UTILISATION POUR LE TRAITEMENT DE MALADIES THROMBOEMBOLIQUES

(30) Priorität: 23.09.2005 DE 102005045518
(43) Veröffentlichungstag der Anmeldung: 11.06.2008
(73) Patentinhaber: Bayer Intellectual Property GmbH, 40789 Monheim (DE)
(72) Erfinder: THOMAS, Christian, R., 42111 Wuppertal (DE); RÖHRIG, Susanne, 40724 Hilden (DE); PERZBORN, Elisabeth, 42327 Wuppertal (DE)
(74) Vertreter: BIP Patents
(86) Internationale Anmeldenummer: PCT/EP2006/008949
(87) Internationale Veröffentlichungsnummer: WO 2007/036306

(56) Entgegenhaltungen:
- WO-A1-01/47919
- DE-A1- 10 105 989

## Beschreibung

Die vorliegende Anmeldung betrifft neue 2-Aminoethoxyessigsäure-Derivate, Verfahren zu ihrer Herstellung sowie ihre Verwendung zur Herstellung von Arzneimitteln zur Behandlung und/oder Prophylaxe von Krankheiten, insbesondere von thromboembolischen Erkrankungen.

Die Blutgerinnung ist ein Schutzmechanismus des Organismus, mit dessen Hilfe Defekte in der Gefäßwand rasch und zuverlässig "abgedichtet" werden können. So kann ein Blutverlust vermieden bzw. minimiert werden. Die Blutstillung nach Gefäßverletzung erfolgt im wesentlichen durch das Gerinnungssystem, bei dem eine enzymatische Kaskade komplexer Reaktionen von Plasmaproteinen ausgelöst wird. Hierbei sind zahlreiche Blutgerinnungsfaktoren beteiligt, von denen jeder, sobald aktiviert, die jeweils nächste inaktive Vorstufe in ihre aktive Form überführt. Am Ende der Kaskade steht die Umwandlung des löslichen Fibrinogens in das unlösliche Fibrin, so dass es zu einem Blutgerinnsel kommt. Traditionell unterscheidet man bei der Blutgerinnung zwischen dem intrinsischen und dem extrinsischen System, die in einem abschließenden gemeinsamen Reaktionsweg münden. Hierbei kommt dem Faktor Xa, der aus dem Proenzym Faktor X gebildet wird, eine Schlüsselrolle zu, da er beide Gerinnungswege verbindet. Die aktivierte Serinprotease Xa spaltet Prothrombin zu Thrombin. Das entstandene Thrombin wiederum spaltet seinerseits Fibrinogen zu Fibrin. Durch anschließende Quervemetzung der Fibrin-Monomere kommt es zur Bildung von Blutgerinnseln und damit zur Blutstillung. Darüber hinaus ist Thrombin ein potenter Auslöser der Thrombozytenaggregation, die ebenfalls einen erheblichen Beitrag bei der Hämostase leistet.

Die Hämostase unterliegt einem komplexen Regulationsmechanismus. Eine unkontrollierte Aktivierung des Gerinnungssystems oder eine defekte Hemmung der Aktivierungsprozesse kann die Bildung von lokalen Thrombosen oder Embolien in Gefäßen (Arterien, Venen, Lymphgefäßen) oder Herzhöhlen bewirken. Dies kann zu schwerwiegenden thromboembolischen Erkrankungen führen. Darüber hinaus kann eine Hyperkoagulabilität - systemisch - bei einer Verbrauchskoagulopathie zur disseminierten intravasalen Gerinnung führen. Thromboembolische Komplikationen treten ferner auf bei mikroangiopathischen hämolytischen Anämien, extrakorporalen Blutkreisläufen, wie Hämodialyse, sowie Herzklappenprothesen.

Thromboembolische Erkrankungen sind die häufigste Ursache von Mörbidität und Mortalität in den meisten industrialisierten Ländern [Heart Disease: A Textbook of Cardiovascular Medicine, Eugene Braunwald, 5. Auflage, 1997, W.B. Saunders Company, Philadelphia].

Die aus dem Stand der Technik bekannten Antikoagulantien, d.h. Stoffe zur Hemmung oder Verhinderung der Blutgerinnung, weisen verschiedene, oftmals gravierende Nachteile auf. Eine effiziente Behandlungsmethode bzw. Prophylaxe von thromboembolischen Erkrankungen erweist sich in der Praxis deshalb als sehr schwierig und unbefriedigend.

Für die Therapie und Prophylaxe von thromboembolischen Erkrankungen findet zum einen Heparin Verwendung, das parenteral oder subkutan appliziert wird. Aufgrund günstigerer pharmakokinetischer Eigenschaften wird zwar heutzutage zunehmend niedermolekulares Heparin bevorzugt; allerdings können auch hierdurch die im folgenden geschilderten bekannten Nachteile nicht vermieden werden, die bei der Therapierung mit Heparin bestehen. So ist Heparin oral unwirksam und besitzt nur eine vergleichsweise geringe Halbwertszeit. Da Heparin gleichzeitig mehrere Faktoren der Blutgerinnungskaskade hemmt, kommt es zu einer unselektiven Wirkung. Darüber hinaus besteht ein hohes Blutungsrisiko, insbesondere können Hirnblutungen und Blutungen im Gastrointestinaltrakt auftreten, und es kann zu Thrombopenie, Alopecia medicomentosa oder Osteoporose kommen [Pschyrembel, Klinisches Wörterbuch, 257. Auflage, 1994, Walter de Gruyter Verlag, Seite 610, Stichwort "Heparin"; Römpp Lexikon Chemie, Version 1.5, 1998, Georg Thieme Verlag Stuttgart, Stichwort "Heparin"].

Eine zweite Klasse von Antikoagulantien stellen die Vitamin K-Antagonisten dar. Hierzu gehören beispielsweise 1,3-Indandione, vor allem aber Verbindungen wie Warfarin, Phenprocoumon, Dicumarol und andere Cumarin-Derivate, die unselektiv die Synthese verschiedener Produkte bestimmter Vitamin K-abhängiger Gerinnungsfaktoren in der Leber hemmen. Durch den Wirkmechanismus bedingt, setzt die Wirkung aber nur sehr langsam ein (Latenzzeit bis zum Wirkeintritt 36 bis 48 Stunden). Die Verbindungen können zwar oral appliziert werden, aufgrund des hohen Blutungsrisikos und des engen therapeutischen Indexes ist aber eine aufwendige individuelle Einstellung und Beobachtung des Patienten notwendig [J. Hirsh, J. Dalen, D.R. Anderson et al., "Oral anticoagulants: Mechanism of action, clinical effectiveness, and optimal therapeutic range" Chest 2001, 119, 8S-21S; J. Ansell, J. Hirsh, J. Dalen et al., "Managing oral anticoagulant therapy" Chest 2001, 119, 22S-38S; P.S. Wells, A.M. Holbrook, N.R Crowther et al., "Interactions of warfarin with drugs and food" Ann. Intern. Med. 1994, 121, 676-683].

In jüngster Zeit ist ein neuer Therapieansatz für die Behandlung und Prophylaxe von thromboembolischen Erkrankungen beschrieben worden. Ziel dieses neuen Therapieansatzes ist die Inhibierung von Faktor Xa. Entsprechend der zentralen Rolle, die Faktor Xa in der Blutgerinnungskaskade spielt, stellt Faktor Xa eines der wichtigsten Targets für antikoagulatorische Wirkstoffe dar [J. Hauptmann, J. Stürzebecher, Thrombosis Research 1999, 93, 203; S.A.V. Raghavan, M. Dikshit, "Recent advances in the status and targets of antithrombotic agents" Drugs Fut. 2002, 27, 669-683; H.A. Wieland, V. Laux, D. Kozian, M. Lorenz, "Approaches in anticoagulation: Rationales for target positioning" Curr. Opin. Investig. Drugs 2003, 4, 264-271; U.J. Ries, W. Wienen, "Serine proteases as targets for antithrombotic therapy" Drugs Fut. 2003, 28, 355-370; L.-A. Linkins, J.I. Weitz, "New anticoagulant therapy" Annu. Rev. Med. 2005, 56, 63-77 (online-Publikation August 2004)].

Dabei ist gezeigt worden, dass verschiedene, sowohl peptidische wie nicht-peptidische Verbindungen in Tiermodellen als Faktor Xa-Inhibitoren wirksam sind. Eine große Anzahl von direkten Faktor Xa-Inhibitoren ist bislang bekannt [J.M. Walenga, W.P. Jeske, D. Hoppensteadt, J. Fareed, "Factor Xa Inhibitors: Today and beyond" Curr. Opin. Investig. Drugs 2003, 4, 272-281; J. Ruef, H.A. Katus, "New antithrombotic drugs on the horizon" Expert Opin. Investig. Drugs 2003, 12, 781-797; M.L. Quan, J.M. Smallheer, "The race to an orally active Factor Xa inhibitor: Recent advances" Curr. Opin. Drug Discovery & Development 2004, 7, 460-469]. Nicht-peptidische Faktor Xa-Inhibitoren mit Oxazolidinon-Kemstruktur werden in WO 01/047919 und WO 02/064575 beschrieben.

Die Aufgabe der vorliegenden Erfindung liegt in der Bereitstellung neuer Substanzen zur Bekämpfung von Erkrankungen, insbesondere von thromboembolischen Erkrankungen, die eine verbesserte Löslichkeit in Wasser und physiologischen Medien aufweisen.

Gegenstand der vorliegenden Erfindung sind Verbindungen der allgemeinen Formel (I) 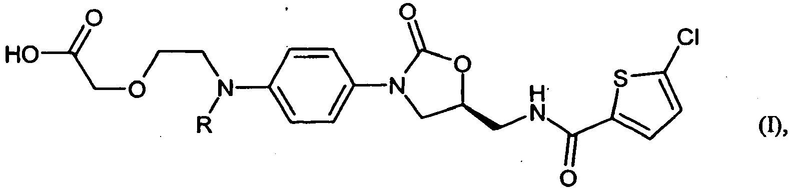 in welcher
- R: für Wasserstoff, (C₁-C₆)-Alkyl, (C₁-C₆)-Alkanoyl, (C₁-C₆)-Alkoxycarbonyl, Mono- oder Di-(C₁-C₆)-alkylaminocarbonyl, Benzoyl oder Heteroaroyl steht, wobei Benzoyl und Heteroaroyl ihrerseits mit Halogen, Cyano, (C₁-C₄)-Alkyl oder (C₁-C₄)-Alkoxy substituiert sein können,
sowie ihre Salze, Solvate und Solvate der Salze.

Erfindungsgemäße Verbindungen sind die Verbindungen der Formel (I) und deren Salze, Solvate und Solvate der Salze, die von Formel (I) umfassten Verbindungen der nachfolgend genannten Formeln und deren Salze, Solvate und Solvate der Salze sowie die von Formel (I) umfassten, nachfolgend als Ausführungsbeispiele genannten Verbindungen und deren Salze, Solvate und Solvate der Salze, soweit es sich bei den von Formel (I) umfassten, nachfolgend genannten Verbindungen nicht bereits um Salze, Solvate und Solvate der Salze handelt.

Die erfindungsgemäßen Verbindungen können in Abhängigkeit von ihrer Struktur in stereoisomeren Formen (Enantiomere, Diastereomere) existieren. Die Erfindung umfasst deshalb die Enantiomeren oder Diastereomeren und ihre jeweiligen Mischungen. Aus solchen Mischungen von Enantiomeren und/oder Diastereomeren lassen sich die stereoisomer einheitlichen Bestandteile in bekannter Weise isolieren.

Sofern die erfindungsgemäßen Verbindungen in tautomeren Formen vorkommen können, umfasst die vorliegende Erfindung sämtliche tautomere Formen.

Als Salze sind im Rahmen der vorliegenden Erfindung physiologisch unbedenkliche Salze der erfindungsgemäßen Verbindungen bevorzugt. Umfasst sind auch Salze, die für pharmazeutische Anwendungen selbst nicht geeignet sind, jedoch beispielsweise für die Isolierung oder Reinigung der erfindungsgemäßen Verbindungen verwendet werden können.

Physiologisch unbedenkliche Salze der erfindungsgemäßen Verbindungen umfassen Säureadditionssalze von Mineralsäuren, Carbonsäuren und Sulfonsäuren, z.B. Salze der Chlorwasserstoffsäure, Bromwasserstoffsäure, Schwefelsäure, Phosphorsäure, Methansulfonsäure, Ethansulfonsäure, Toluolsulfonsäure, Benzolsulfonsäure, Naphthalindisulfonsäure, Essigsäure, Trifluoressigsäure, Propionsäure, Milchsäure, Weinsäure, Äpfelsäure, Zitronensäure, Fumarsäure, Maleinsäure und Benzoesäure.

Physiologisch unbedenkliche Salze der erfindungsgemäßen Verbindungen umfassen auch Salze üblicher Basen, wie beispielhaft und vorzugsweise Alkalimetallsalze (z.B. Natrium- und Kaliumsalze), Erdalkalisalze (z.B. Calcium- und Magnesiumsalze) und Ammoniumsalze, abgeleitet von Ammoniak oder organischen Aminen mit 1 bis 16 C-Atomen, wie beispielhaft und vorzugsweise Ethylamin, Diethylamin, Triethylamin, Ethyldiisopropylamin, Monoethanolamin, Diethanolamin, Triethanolamin, Dicyclohexylamin, Dimethylaminoethanol, Prokain, Dibenzylamin, N-Methylmorpholin, Arginin, Lysin, Ethylendiamin und N-Methylpiperidin.

Als Solvate werden im Rahmen der Erfindung solche Formen der erfindungsgemäßen Verbindungen bezeichnet, welche in festem oder flüssigem Zustand durch Koordination mit Lösungsmittelmolekülen einen Komplex bilden. Hydrate sind eine spezielle Form der Solvate, bei denen die Koordination mit Wasser erfolgt. Als Solvate sind im Rahmen der vorliegenden Erfindung Hydrate bevorzugt.

Außerdem umfasst die vorliegende Erfindung auch Prodrugs der erfindungsgemäßen Verbindungen. Der Begriff "Prodrugs" umfasst Verbindungen, welche selbst biologisch aktiv oder inaktiv sein können, jedoch während ihrer Verweilzeit im Körper zu erfindungsgemäßen Verbindungen umgesetzt werden (beispielsweise metabolisch oder hydrolytisch).

Im Rahmen der vorliegenden Erfindung haben die Substituenten, soweit nicht anders spezifiziert, die folgende Bedeutung:
(C₁-C₆)₋Alkyl und (C₁₋C₄)-Alkyl stehen im Rahmen der Erfindung für einen geradkettigen oder verzweigten Alkylrest mit 1 bis 6 bzw. 1 bis 4 Kohlenstoffatomen. Bevorzugt ist ein geradkettiger oder verzweigter Alkylrest mit 1 bis 4 Kohlenstoffatomen. Beispielhaft und vorzugsweise seien genannt: Methyl, Ethyl, n-Propyl, Isopropyl, n-Butyl, iso-Butyl, sec.-Butyl, tert.-Butyl, 1-Ethylpropyl, n-Pentyl und n-Hexyl.
(C₁-C₆)-Alkoxy und (C₁-C₄)-Alkoxy stehen im Rahmen der Erfindung für einen geradkettigen oder verzweigten Alkoxyrest mit 1 bis 6 bzw. 1 bis 4 Kohlenstoffatomen. Bevorzugt ist ein geradkettiger oder verzweigter Alkoxyrest mit 1 bis 4 Kohlenstoffatomen. Beispielhaft und vorzugsweise seien genannt: Methoxy, Ethoxy, n-Propoxy, Isopropoxy, n-Butoxy und tert.-Butoxy.
(C₁-C₆ -Alkanoyl [(C₁-C₆)-Acyl] steht im Rahmen der Erfindung für einen geradkettigen oder verzweigten Alkylrest mit 1. bis 6 Kohlenstoffatomen, der in der 1-Position ein doppelt gebundenes Sauerstoffatom trägt und über die 1-Position verknüpft ist. Bevorzugt ist ein geradkettiger oder verzweigter Alkanoylrest mit 1 bis 4 Kohlenstoffatomen. Beispielhaft und vorzugsweise seien genannt: Formyl, Acetyl, Propionyl, n-Butyryl, iso-Butyryl und Pivaloyl.
(C₁-C₆)-Alkoxycarbonyl steht im Rahmen der Erfindung für einen geradkettigen oder verzweigten Alkoxyrest mit 1 bis 6 Kohlenstoffatomen, der über eine Carbonylgruppe verknüpft ist. Bevorzugt ist ein geradkettiger oder verzweigter Alkoxycarbonylrest mit 1 bis 4 Kohlenstoffatomen in der Alkoxy-Gruppe. Beispielhaft und vorzugsweise seien genannt: Methoxycarbonyl, Ethoxycarbonyl, n-Propoxycarbonyl, Isopropoxycarbonyl und tert.-Butoxycarbonyl.
Mono-C₁-C₆-alkylamino steht im Rahmen der Erfindung für eine Amino-Gruppe mit einem geradkettigen oder verzweigten Alkylsubstituenten, der 1 bis 6 Kohlenstoffatome aufweist. Bevorzugt ist ein geradkettiger oder verzweigter Monoalkylamino-Rest mit 1 bis 4 Kohlenstoffatomen. Beispielhaft und vorzugsweise seien genannt: Methylamino, Ethylamino, n-Propylamino, Isopropylamino, n-Butylamino, Isobutylamino und tert.-Butylamino.
Di-(C₁-C₆)-alkylamino steht im Rahmen der Ereindung für eine Amino-Gruppe mit zwei gleichen oder verschiedenen geradkettigen oder verzweigten Alkylsubstituenten, die jeweils 1 bis 6 Kohlenstoffatome aufweisen. Bevorzugt sind geradkettige oder verzweigte Dialkylamino-Reste mit jeweils 1 bis 4 Kohlenstoffatomen. Beispielhaft und vorzugsweise seien genannt: *N,N* Dimethylamino, *N,N*-Diethylamino, *N*-Ethyl-*N*-methylamino, *N*-Methyl-*N*-n-propylamino, *N*-Isopropyl-*N*-n-propylamino, *N*-tert.-Butyl-*N*-methylamino, *N*-Ethyl-*N*-n-pentylamino und *N*-n-Hexyl-*N*-methylamino.
Mono-(C₁-C₆)-alkylaminocarbonyl und Mono"(C₁-C₄)-alkylaminocarbonyl stehen im Rahmen der Erfindung für einen geradkettigen oder verzweigten Monoalkylamino-Rest mit 1 bis 6 bzw. 1 bis 4 Kohlenstoffatomen, der über eine Carbonylgruppe verknüpft ist. Bevorzugt ist ein geradkettiger oder verzweigter Monoalkylaminocarbonyl-Rest mit 1 bis 4 Kohlenstoffatomen in der Alkylamino-Gruppe. Beispielhaft und vorzugsweise seien genannt: Methylaminocarbonyl, Ethylaminocarbonyl, n-Propylaminocarbonyl, Isopropylaminocarbonyl, n-Butylaminocarbonyl, Isobutylaminocarbonyl und tert.-Butylaminocarbonyl.
Di₋(C₁₋C₆)-alkylaminocarbonyl steht im Rahmen der Erfindung für einen geradkettigen oder verzweigten Dialkylamino-Rest mit jeweils 1 bis 6 Kohlenstoffatomen, der über eine Carbonylgruppe verknüpft ist. Bevorzugt sind geradkettige oder verzweigte Dialkylaminocarbonyl-Reste mit jeweils 1 bis 4 Kohlenstoffatomen in der Alkylamino-Gruppe. Beispielhaft und vorzugsweise seien genannt: *N,N*-Dimethylaminocarbonyl, *N,N-*Diethylaminocarbonyl, *N*-Ethyl-*N*-methylaminocarbonyl, *N*-Methyl-*N*-n-propylaminocarbonyl, *N*-Isopropyl-*N*-n-propylaininocarbonyl, *N*-tert.-Butyl-*N-*methylaminocarbonyl, *N*-Ethyl-*N*-n-pentylaminocarbonyl und *N*-n-Hexyl-*N*-methylaminocarbonyl.
Heteroaroyl (Heteroarylcarbonyl) steht im Rahmen der Erfindung für einen aromatischen Heterocyclus (Heteroaromaten) mit insgesamt 5 oder 6 Ringatomen und bis zu drei gleichen oder verschiedenen Ring-Heteroatomen aus der Reihe N, O und/oder S, der über eine Carbonylgruppe verknüpft ist. Beispielhaft seien genannt: Furoyl, Pyrroyl, Thienoyl, Pyrazoyl, Imidazoyl, Thiazoyl, Oxazoyl, Isoxazoyl, Isothiazoyl, Triazoyl, Oxadiazoyl, Thiadiazoyl, Pyridinoyl, Pyrimidinoyl, Pyridazinoyl, Pyrazinoyl. Bevorzugt ist ein 5- oder 6-gliedriger Heteroaroyl-Rest mit bis zu zwei Heteroatomen aus der Reihe N, O und/oder S wie beispielsweise Furoyl, Thienoyl, Thiazoyl, Oxazoyl, Isoxazoyl, Isothiazoyl, Pyridinoyl, Pyrimidinoyl, Pyridazinoyl, Pyrazinoyl.
Halogen schließt im Rahmen der Erfindung Fluor, Chlor, Brom und Iod ein. Bevorzugt sind Chlor oder Fluor.

Wenn Reste in den erfindungsgemäßen Verbindungen substituiert sind, können die Reste, soweit nicht anders spezifiziert, ein- oder mehrfach substituiert sein. Im Rahmen der vorliegenden Erfindung gilt, dass für alle Reste, die mehrfach auftreten, deren Bedeutung unabhängig voneinander ist. Eine Substitution mit ein, zwei oder drei gleichen oder verschiedenen Substituenten ist bevorzugt. Ganz besonders bevorzugt ist die Substitution mit einem Substituenten.

Bevorzugt sind Verbindungen der Formel (I), in welcher
- R: für Wasserstoff, Methyl, Acetyl oder für Thienylcarbonyl, das mit Chlor substituiert sein kann, steht,
sowie ihre Salze, Solvate und Solvate der Salze.

Ebenfalls bevorzugt sind Verbindungen der Formel (I), in welcher
- R: für Mono-(C₁-C₄)-alkylaminocarbonyl steht,
sowie ihre Salze, Solvate und Solvate der Salze.

Besonders bevorzugt sind Verbindungen der Formel (I), in welcher
- R: für Wasserstoff, Isobutylaminocarbonyl oder 5-Chlor-2-thienylcarbonyl steht,
sowie ihre Salze, Solvate und Solvate der Salze.

Ganz besonders bevorzugt ist die Verbindung gemäß Formel (I) mit folgender Struktur: 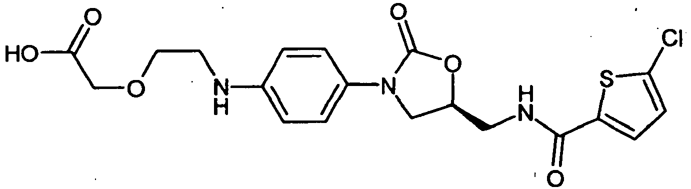 sowie ihre Salze, Solvate und Solvate der Salze.

Weiterer Gegenstand der Erfindung ist ein Verfahren zur Herstellung der erfindungsgemäßen Verbindungen der Formel (I), dadurch gekennzeichnet, dass man die Verbindung der Formel (II) 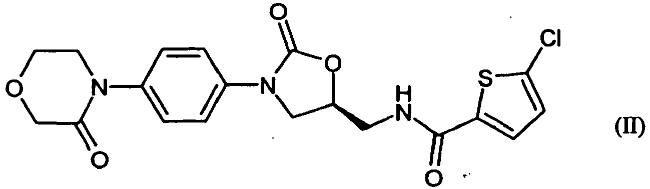 durch selektive Hydrolyse in die Verbindung der Formel (I-A) 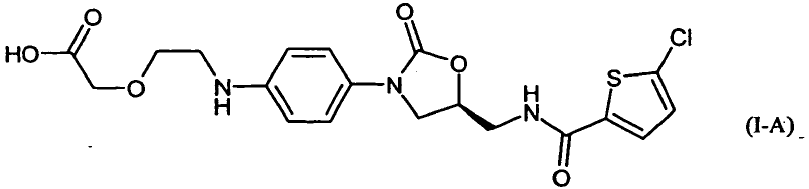 überführt und diese dann in einem inerten Lösungsmittel, gegebenenfalls in Gegenwart einer Base, mit einer Verbindung der Formel (III) 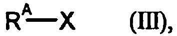 in welcher
- R^{A}: für (C₁-C₆)-Alkyl, (C₁-C₆)-Alkanoyl, (C₁-C₆)-Alkoxycarbonyl, Di-(C₁-C₆)-alkylaminocarbonyl, Benzoyl oder Heteroaroyl steht, wobei Benzoyl und Heteroaroyl ihrerseits mit Halogen, Cyano, (C₁-C₄)-Alkyl oder (C₁-C₄)-Alkoxy substituiert sein können,
und
- X: für eine Abgangsgruppe wie beispielsweise Halogen steht,
oder im Falle, dass R für Mono-(C₁-C₆)-alkylaminocarbonyl steht, mit einer Verbindung der Formel (IV) 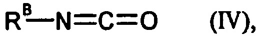 in welcher
- R^{B}: für (C₁-C₆)-Alkyl steht,
umsetzt
und die resultierenden Verbindungen der Formel (I) bzw. (I-A) gegebenenfalls mit den entsprechenden (i) Lösungsmitteln und/oder (ii) Basen oder Säuren in ihre Solvate, Salze und/oder Solvate der Salze überführt.

Die Hydrolyse im Verfahrensschritt (II) → (I-A) wird vorteilhafterweise unter sauren Bedingungen durchgeführt. Bevorzugt wird hierfür ein Gemisch aus Essigsäure und Salzsäure verwendet. Die Reaktion wird in einem Temperaturbereich von +50°C bis +100°C, bevorzugt bei +70°C durchgeführt. Die Umsetzung kann bei normalem, erhöhtem oder erniedrigtem Druck erfolgen (z.B. bei 0.5 bis 5 bar). Im Allgemeinen arbeitet man bei Normaldruck.

Inerte Lösungsmittel für den Verfahrensschritt (I-A) + (III) bzw. (IV) → (I) sind beispielsweise Ether wie Diethylether, tert.-Butylmethylether, Dioxan, Tetrahydrofuran, Glykoldimethylether oder Diethylenglykoldimethylether, Kohlenwasserstoffe wie Benzol, Toluol, Xylol, Hexan, Cyclohexan oder Erdölfraktionen, Halogenkohlenwasserstoffe wie Dichlormethan, Trichlormethan, Tetrachlormethan, 1,2-Dichlorethan, Trichlorethylen oder Chlorbenzol, oder andere Lösungsmittel wie Ethylacetat, Aceton, Acetonitril, Pyridin, Dimethylsulfoxid, Dimethylformamid, *N,N'-*Dimethylpropylenharnstoff (DMPU), *N*-Methylpyrrolidon (NMP) oder gegebenenfalls auch Wasser. Ebenso ist es möglich, Gemische der genannten Lösungsmittel zu verwenden. Bevorzugt sind Dichlormethan, Tetrahydrofuran, Dimethylformamid, Aceton, Wasser oder Gemische dieser Lösungsmittel.

Der Verfahrensschritt (I-A) + (III) bzw. (IV) → (I) kann vorteilhafterweise in Gegenwart einer Base durchgeführt werden. Hierfür eignen sich die üblichen anorganischen oder organischen Basen. Dazu gehören bevorzugt Alkalihydroxide wie beispielsweise Lithium-, Natrium- oder Kaliumhydroxid, Alkalihydrogencarbonate wie Natrium- oder Kaliumhydrogencarbonat, Alkali-oder Erdalkalicarbonate wie Lithium-, Natrium-, Kalium-, Calcium- oder Cäsiumcarbonat, Alkalihydride wie Natriumhydrid, Amide wie Lithium- oder Kalium-bis(trimethylsilyl)amid oder Lithiumdiisopropylamid, oder organische Amine wie Triethylamin, *N*-Methylmorpholin, *N-*Methylpiperidin, *N,N*-Diisopropylethylamin, Pyridin, 1,5-Diazabicyclo[4.3.0]non-5-en (DBN), 1,4-Diazabicyclo[2.2.2]octan (DABCO^{®}) oder 1,8-Diazabicyclo[5.4.0]undec-7-en (DBU). Besonders bevorzugt sind Natrium-, Kalium- oder Cäsiumcarbonat, Triethylamin, *N,N*-Diisopropylethylamin oder Pyridin.

Die Reaktion (I-A) + (III) bzw. (IV) → (I) wird vorzugsweise in einem Temperaturbereich von 0°C bis +50°C durchgeführt. Die Umsetzung kann bei normalem, erhöhtem oder erniedrigtem Druck erfolgen (z.B. bei 0.5 bis 5 bar). Im Allgemeinen arbeitet man bei Normaldruck.

Die Verbindung der Formel (II) und ihre Herstellung ist in WO 01/047919 (Beispiel 44) beschrieben. Die Verbindungen der Formeln (III) und (IV) sind kommerziell erhältlich, literaturbekannt oder können in Analogie zu literaturbekannten Verfahren hergestellt werden.

Die Herstellung der erfindungsgemäßen Verbindungen kann durch das folgende Syntheseschema veranschaulicht werden:

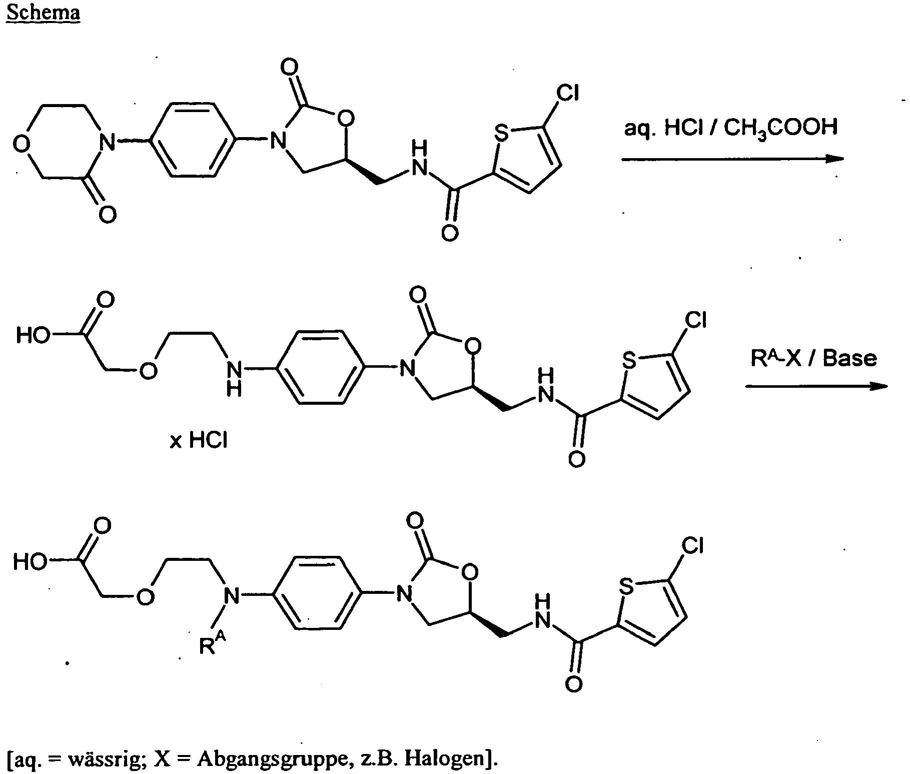

Die erfindungsgemäßen Verbindungen zeigen ein nicht vorhersehbares, wertvolles pharmakologisches Wirkspektrum. Sie eignen sich daher zur Verwendung als Arzneimittel zur Behandlung und/oder Prophylaxe von Krankheiten bei Menschen und Tieren.

Bei den erfindungsgemäßen Verbindungen handelt es sich um selektive Inhibitoren des Blutgerinnungsfaktors Xa, die insbesondere als Antikoagulantien wirken. Darüber hinaus verfügen die erfindungsgemäßen Verbindungen über günstige physikochemische Eigenschaften, wie beispielsweise eine gute Löslichkeit in Wasser und physiologischen Medien, was für ihre therapeutische Anwendung von Vorteil ist.

Weiterer Gegenstand der vorliegenden Erfindung ist der Einsatz der erfindungsgemäßen Verbindungen zur Behandlung und/oder Prophylaxe von Erkrankungen, vorzugsweise von thromboembolischen Erkrankungen und/oder thromboembolischen Komplikationen.

Zu den "thromboembolischen Erkrankungen" im Sinne der vorliegenden Erfindung zählen insbesondere Erkrankungen wie Herzinfarkt mit ST-Segment-Erhöhung (STEMI) und ohne ST-Segment-Erhöhung (non-STEMI), stabile Angina Pectoris, instabile Angina Pectoris, Reokklusionen und Restenosen nach Koronarinterventionen wie Angioplastie oder aortokoronarem Bypass, periphere arterielle Verschlusskrankheiten, Lungenembolien, tiefe venöse Thrombosen und Nierenvenenthrombosen, transitorische ischämische Attacken sowie thrombotischer und thromboembolischer Hirnschlag.

Die Substanzen eignen sich daher auch zur Prävention und Behandlung von kardiogenen Thromboembolien, wie beispielsweise Hirn-Ischämien, Schlaganfall und systemischen Thromboembolien und Ischämien, bei Patienten mit akuten, intermittierenden oder persistierenden Herzarrhythmien, wie beispielsweise Vorhofflimmern, und solchen, die sich einer Kardioversion unterziehen, ferner bei Patienten mit Herzklappen-Erkrankungen oder mit künstlichen Herzklappen. Darüber hinaus sind die erfindungsgemäßen Verbindungen zur Behandlung der disseminierten intravasalen Gerinnung (DIC) geeignet.

Thromboembolische Komplikationen treten ferner auf bei mikroangiopathischen hämolytischen Anämien, extrakorporalen Blutkreisläufen, wie Hämodialyse, sowie Herzklappenprothesen.

Außerdem kommen die erfindungsgemäßen Verbindungen auch für die Prophylaxe und/oder Behandlung von atherosklerotischen Gefäßerkrankungen und entzündlichen Erkrankungen wie rheumatische Erkrankungen des Bewegungsapparats in Betracht, darüber hinaus ebenso für die Prophylaxe und/oder Behandlung der Alzheimer'schen Erkrankung. Außerdem können die erfindungsgemäßen Verbindungen zur Inhibition des Tumorwachstums und der Metastasenbildung, bei Mikroangiopathien, altersbedingter Makula-Degeneration, diabetischer Retinopathie, diabetischer Nephropathie und anderen mikrovaskulären Erkrankungen sowie zur Prävention und Behandlung thromboembolischer Komplikationen, wie beispielsweise venöser Thromboembolien, bei Tumorpatienten, insbesondere solchen, die sich größeren chirurgischen Eingriffen oder einer Chemo-oder Radiotherapie unterziehen, eingesetzt werden.

Die erfindungsgemäßen Verbindungen können darüber hinaus auch zur Verhinderung von Koagulation *ex vivo* eingesetzt werden, z.B. zur Konservierung von Blut- und Plasmaprodukten, zur Reinigung/Vorbehandlung von Kathetern und anderen medizinischen Hilfsmitteln und Geräten, zur Beschichtung künstlicher Oberflächen von *in vivo* oder *ex vivo* eingesetzten medizinischen Hilfsmitteln und Geräten oder bei biologischen Proben, die Faktor Xa enthalten.

Weiterer Gegenstand der vorliegenden Erfindung ist die Verwendung der erfindungsgemäßen Verbindungen zur Herstellung eines Arzneimittels zur Behandlung und/oder Prophylaxe von Erkrankungen, insbesondere der zuvor genannten Erkrankungen.

Weiterer Gegenstand der vorliegenden Erfindung ist ein Verfahren zur Verhinderung der Blutkoagulation *in vitro,* insbesondere bei Blutkonserven oder biologischen Proben, die Faktor Xa enthalten, das dadurch gekennzeichnet ist, dass eine antikoagulatorisch wirksame Menge der erfindungsgemäßen Verbindung zugegeben wird.

Weiterer Gegenstand der vorliegenden Erfindung sind Arzneimittel, enthaltend eine erfindungsgemäße Verbindung und einen oder mehrere weitere Wirkstoffe, insbesondere zur Behandlung und/oder Prophylaxe der zuvor genannten Erkrankungen. Als geeignete Kombinationswirkstoffe seien beispielhaft und vorzugsweise genannt:
- Lipidsenker, insbesondere HMG-CoA-(3-Hydroxy-3-methylglutaryl-Coenzym A)-Reduktase-Inhibitoren;
- Koronartherapeutika/Vasodilatatoren, insbesondere ACE-(Angiotensin-Converting-Enzyme)-Inhibitoren; AII-(Angiotensin II)-Rezeptor-Antagonisten; β-Adrenozeptor-Antagonisten; alpha-1-Adrenozeptor-Antagonisten; Diuretika; Calciumkanal-Blocker; Substanzen, die eine Erhöhung von cyclischem Guanosinmonophosphat (cGMP) bewirken, wie beispielsweise Stimulatoren der löslichen Guanylatcyclase;
- Plasminogen-Aktivatoren (Thrombolytika/Fibrinolytika) und die Thrombolyse/Fibrinolyse steigernde Verbindungen wie Inhibitoren des Plasminogen-Aktivator-Inhibitors (PAI-Inhibitoren) oder Inhibitoren des Thrombin-aktivierten Fibrinolyse-Inhibitors (TAFI-Inhibitoren);
- antikoagulatorisch wirksame Substanzen (Antikoagulantien);
- plättchenaggregationshemmende Substanzen (Plättchenaggregationshemmer, Thrombozytenaggregationshemmer);
- Fibrinogen-Rezeptor-Antagonisten (Glycoprotein-Ilb/II1a-Antagonisten);
- sowie Antiarrhythmika.

Weiterer Gegenstand der vorliegenden Erfindung sind Arzneimittel, die mindestens eine erfindungsgemäße Verbindung, üblicherweise zusammen mit einem oder mehreren inerten, nicht-toxischen, pharmazeutisch geeigneten Hilfsstoffen enthalten, sowie deren Verwendung zu den zuvor genannten Zwecken.

Die erfindungsgemäßen Verbindungen können systemisch und/oder lokal wirken. Zu diesem Zweck können sie auf geeignete Weise appliziert werden, wie z.B. oral, parenteral, pulmonal, nasal, sublingual, lingual, buccal, rectal, dermal, transdermal, conjunctival, otisch oder als Implantat bzw. Stent.

Für diese Applikationswege können die erfindungsgemäßen Verbindungen in geeigneten Applikationsformen verabreicht werden.

Für die orale Applikation eignen sich nach dem Stand der Technik funktionierende, die erfindungsgemäßen Verbindungen schnell und/oder modifiziert abgebende Applikationsformen, die die erfindungsgemäßen Verbindungen in kristalliner und/oder amorphisierter und/oder gelöster Form enthalten, wie z.B. Tabletten (nicht-überzogene oder überzogene Tabletten, beispielsweise mit magensaftresistenten oder sich verzögert auflösenden oder unlöslichen Überzügen, die die Freisetzung der erfindungsgemäßen Verbindung kontrollieren), in der Mundhöhle schnell zerfallende Tabletten oder Filme/Oblaten, Filme/Lyophylisate, Kapseln (beispielsweise Hart- oder Weichgelatinekapseln), Dragees, Granulate, Pellets, Pulver, Emulsionen, Suspensionen, Aerosole oder Lösungen.

Die parenterale Applikation kann unter Umgehung eines Resorptionsschrittes geschehen (z.B. intravenös, intraarteriell, intrakardial, intraspinal oder intralumbal) oder unter Einschaltung einer Resorption (z.B. intramuskulär, subcutan, intracutan, percutan oder intraperitoneal). Für die parenterale Applikation eignen sich als Applikationsformen u.a. Injektions- und Infusionszubereitungen in Form von Lösungen, Suspensionen, Emulsionen, Lyophilisaten oder sterilen Pulvern.

Für die sonstigen Applikationswege eignen sich z.B. Inhalationsarzneiformen (u.a. Pulverinhalatoren, Nebulizer), Nasentropfen, -lösungen oder -sprays, lingual, sublingual oder buccal zu applizierende Tabletten, Filme/Oblaten oder Kapseln, Suppositorien, Ohren- oder Augenpräparationen, Vaginalkapseln, wässrige Suspensionen (Lotionen, Schüttelmixturen), lipophile Suspensionen, Salben, Cremes, transdermale therapeutische Systeme (z.B. Pflaster), Milch, Pasten, Schäume, Streupuder, Implantate oder Stents.

Bevorzugt sind die orale oder parenterale Applikation, insbesondere die orale Applikation.

Die erfindungsgemäßen Verbindungen können in die angeführten Applikationsformen überführt werden. Dies kann in an sich bekannter Weise durch Mischen mit inerten, nichttoxischen, pharmazeutisch geeigneten Hilfsstoffen geschehen. Zu diesen Hilfsstoffen zählen u.a. Trägerstoffe (beispielsweise mikrokristalline Cellulose, Lactose, Mannitol), Lösungsmittel (z.B. flüssige Polyethylenglycole), Emulgatoren und Dispergier- oder Netzmittel (beispielsweise Natriumdodecylsulfat, Polyoxysorbitanoleat), Bindemittel (beispielsweise Polyvinylpyrrolidon), synthetische und natürliche Polymere (beispielsweise Albumin), Stabilisatoren (z.B. Antioxidantien wie beispielsweise Ascorbinsäure), Farbstoffe (z.B. anorganische Pigmente wie beispielsweise Eisenoxide) und Geschmacks- und/oder Geruchskorrigentien.

Im Allgemeinen hat es sich als vorteilhaft erwiesen, bei parenteraler Applikation Mengen von etwa 0.001 bis 1 mg/kg, vorzugsweise etwa 0.01 bis 0.5 mg/kg Körpergewicht zur Erzielung wirksamer Ergebnisse zu verabreichen. Bei oraler Applikation beträgt die Dosierung etwa 0.01 bis 100 mg/kg, vorzugsweise etwa 0.01 bis 20 mg/kg und ganz besonders bevorzugt 0.1 bis 10 mg/kg Körpergewicht.

Trotzdem kann es gegebenenfalls erforderlich sein, von den genannten Mengen abzuweichen, und zwar in Abhängigkeit von Körpergewicht, Applikationsweg, individuellem Verhalten gegenüber dem Wirkstoff, Art der Zubereitung und Zeitpunkt bzw. Intervall, zu welchem die Applikation erfolgt. So kann es in einigen Fällen ausreichend sein, mit weniger als der vorgenannten Mindestmenge auszukommen, während in anderen Fällen die genannte obere Grenze überschritten werden muss. Im Falle der Applikation größerer Mengen kann es empfehlenswert sein, diese in mehreren Einzelgaben über den Tag zu verteilen.

Die nachfolgenden Ausführungsbeispiele erläutern die Erfindung. Die Erfindung ist nicht auf die Beispiele beschränkt:

Die Prozentangaben in den folgenden Tests und Beispielen sind, sofern nicht anders angegeben, Gewichtsprozente; Teile sind Gewichtsteile. Lösungsmittelverhältnisse; Verdünnungsverhältnisse und Konzentrationsangaben von flüssig/flüssig-Lösungen beziehen sich jeweils auf das Volumen.

### A. Beispiele

### Abkürzungen und Akronyme:

- DMSO: Dimethylsulfoxid
- d. Th.: der Theorie (bei Ausbeute)
- ESI: Elektrospray-Ionisation (bei MS)
- h: Stunde(n)
- HPLC: Hochdruck-, Hochleistungsflüssigchromatographie
- LC-MS: Flüssigchromatographie-gekoppelte Massenspektrometrie
- min: Minute(n)
- MS: Massenspektrometrie
- NMR: Kernresonanzspektrometrie
- RT: Raumtemperatur
- Rₜ: Retentionszeit

### HPLC-Methode:

Hochdruckflüssigkeitschromatograph mit thermostatisiertem Säulenofen, UV-Detektor und Datenauswertesystem; Säule: Cosmosil 5C18-AR-II 5 µm, 25 cm x 4.6 mm; Eluent A: 1.36 g Kaliumdihydrogenphosphat in Wasser zu 1 Liter auffüllen und mit ortho-Phosphorsäure (85%-ig) auf pH 2.1. einstellen; Eluent B: Methanol; Gradient: 0 min 30% B → 35 min 90% B → 40 min 90% B; Durchflussrate: 1 ml/min; Temperatur des Säulenofens: 45°C; UV-Detektion: 250 nm; Injektionsvolumen: 5.0 µl (Prüflösung: 25 mg Probe in 50 ml Acetonitril).

### LC-MS-Methode:

Instrument: Micromass Quattro LCZ mit HPLC Agilent Serie 1100; Säule: Phenomenex Onyx Monolithic C18, 100 mm x 3 mm; Eluent A: 11 Wasser + 0.5 ml 50%-ige Ameisensäure, Eluent B: 11 Acetonitril + 0.5 ml 50%-ige Ameisensäure; Gradient: 0.0 min 90% A →2 min 65% A → 4.5 min 5% A → 6 min 5% A; Fluss: 2 ml/min; Ofen: 40°C; UV-Detektion: 208-400 nm.

### Ausgangsverbindungen und Intermediate:

### Beispiel 1A

### 5-Chlor-N-({(5S)-2-oxo-3-[4-(3-oxomorpholin-4-yl)phenyl]-1,3-oxazolidin-5-yl}methyl)thiophen-2-carboxamid

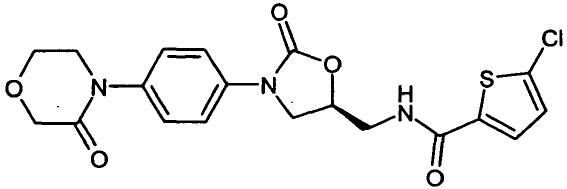

Die Darstellung der Titelverbindung erfolgt auf dem in WO 01/047919 *(*Chem. Abstr. 2001, 135, 92625) unter Beispiel 44 beschriebenen Weg.

### Beispiel 2A

### 5-Chlorthiophen-2-carbonsäurechlorid

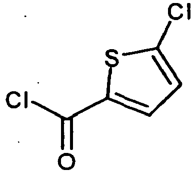

Die Darstellung der Titelverbindung erfolgt durch Umsetzung von 5-Chlorthiophen-2-carbonsäure mit Thionylchlorid, siehe R. Aitken et al., Arch. Pharm. (Weinheim Ger.) 1998, 331, 405-411*.*

### Ausrührunesbeispiele:

### Beispiel 1

### 2-({4-[(5S)-5-({[(5-Chlor-2-thienyl)carbonyl]amino}methyl)-2-oxo-1,3-oxazolidin-3-yl]phenyl}-amino)ethoxy]essigsäure-Hydrochlorid

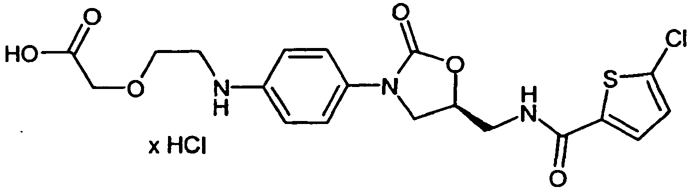

50 g (115 mmol) 5-Chlor-*N*-({(5*S*)-2-oxo-3-[4-(3-oxomorpholin-4-yl)phenyl]-1,3-oxazolidin-5-yl}-methyl)-thiophen-2-carboxamid werden in 100 g Essigsäure, 50 g Wasser und 300 g 37%-iger Salzsäure suspendiert und auf 70°C erhitzt. Das Reaktionsgemisch wird 5-6 h bei 70°C gerührt, wobei nach ca. 2 h eine klare Lösung entsteht. Danach wird auf RT abgekühlt und die entstandene Suspension 15 h bei RT stehen gelassen. Die Kristalle werden abgesaugt und mit 40 ml Essigsäure gewaschen. Zur weiteren Aufreinigung werden die Kristalle zweimal in je 150 ml Isopropanol suspendiert und abgesaugt, dann zweimal mit je 200 ml Isopropanol gewaschen. Die restfeuchten Kristalle werden für 15 h bei 35°C und einem Druck von <80 mbar getrocknet.

Ausbeute: 43 g (76% d. Th.)

HPLC: Rₜ = 12.74 min;

MS (ESI): m/z = 454 [M+H]⁺;

¹H-NMR (500 MHz, DMSO-d₆): δ = 3.39 (m, 2H), 3.60 (m, 2H), 3.71 (m, 2H), 3.85 (m, 1H), 4.10 (s, 2H), 4.1 (m, 1H), 4.82 (m, 1H), 7.20 (d, 1H), 7.27 (br. m, 2H), 7.53 (m, 2H), 7.74 (d, 1H), 9.01 (m, 1H).

### Beispiel 2

### 2-({4-[(5S)-5-({[(5-Chlor-2-thienyl)carbonyl]amino}methyl)-2-oxo-1,3-oxazolidin-3-yl]phenyl}-amino)ethoxy]essigsäure

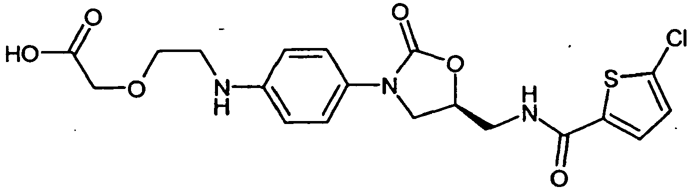

Die Neutralverbindung zu Beispiel 1 lässt sich herstellen, indem man die dort erhaltene wässrige Rohprodukt-Lösung mit Triethylamin auf pH 7-8 stellt, wiederholt mit Dichlormethan extrahiert und durch Zugabe von wenig Essigsäure das Produkt ausfällt. Nach Einengen wird dann der Rückstand aus Methanol/*tert*.-Butylmethylether kristallisiert, mit tert.-Butylmethylether gewaschen und getrocknet.

### Beispiel 3

### [2-([(5-Chlor-2-thienyl)carbonyl]{4-((5S)-5-({[(5-chlor-2-thienyl)carbonyl]amino}methyl)-2-oxo-1,3-oxazolidin-3-yl]phenyl}amino)ethoxy]essigsäure

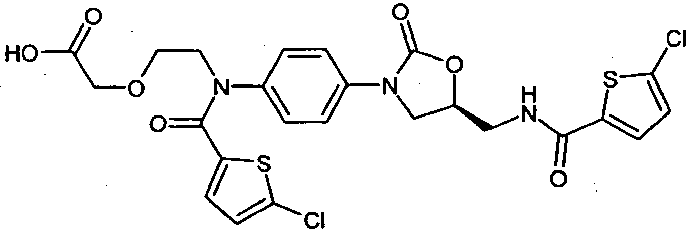

Eine Suspension von 147 mg (0.30 mmol) 2-({4-[(5*S*)-5-({[(5-Chlor-2-thienyl)carbonyl]amino}-methyl)-2-oxo-1,3-oxazolidin-3-yl]phenyl}amino)ethoxy]essigsäure-Hydrochlorid in 1.5 ml Wasser wird bei Raumtemperatur mit 87 mg (0.63 mmol, 2.1 eq.) Kaliumcarbonat versetzt, wobei sich eine Lösung bildet. Das Reaktionsgemisch wird anschließend bei Raumtemperatur tropfenweise mit einer Lösung von 60 mg (0.33 mmol, 1.1 eq.) 5-Chlorthiophen-2-carbonsäurechlorid in 1.5 ml Aceton versetzt und 1 h gerührt. Das Aceton wird danach im Vakuum entfernt und der wässrige Rückstand mit konzentrierter Salzsäure auf pH 1 gestellt. Der entstandene Niederschlag wird abfiltriert, mit Wasser gewaschen und im Vakuum getrocknet.

Ausbeute: 145 mg (81% d. Th.)

HPLC: Rₜ = 25.93 min;

MS (ESI): m/z = 598 [M+H]⁺;

¹H-NMR (400 MHz, DMSO-d₆): δ = 8.99 (t, 1H), 7.70 (d, 1H), 7.63 (d, 2H), 7.45 (d, 2H), 7.19 (d, 1H), 6.93 (d, 1H), 6.51 (d, 1H), 4.91-4.80 (m, 1H), 4.22 (t, 1H), 3.98 (s,-2H), 3.92-3.84 (m, 3H), 3.67-3.59 (m, 4H).

### Beispiel 4

### {2-[{4-[(5S)-5-({[(5-Chlor-2-thienyl)carbonyl]amino}methyl)-2-oxo-1,3-oxazolidin-3-yl]phenyl}-(isobutylcarbamoyl)amino]ethoxy}essigsäure

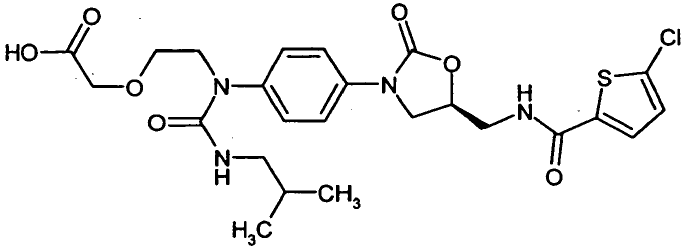

Eine Suspension von **98** mg (0.20 mmol) 2-({4-[(5*S*)-5-({[(5-Chlor-2-thienyl)carbonyl]amino}-methyl)-2-oxo-1,3-oxazolidin-3-yl]phenyl}amino)ethoxy]essigsäure-Hydrochlorid in 4 ml Tetrahydrofuran wird bei Raumtemperatur mit 38 µl (0.22 mmol, 1.1 eq.) *N,N*-Diisopropylethylamin versetzt, wobei sich eine Lösung bildet. Das Reaktionsgemisch wird anschließend bei Raumtemperatur tropfenweise mit 22 mg (0.22 mmol, 1.1 eq.) 1-Isocyanato-2-methylpropan versetzt und über Nacht gerührt. Nach Zugabe von Wasser und Ethylacetat sowie Phasentrennung wird die wässrige Phase mehrmals mit Ethylacetat nachextrahiert. Die vereinigten organischen Phasen werden über Natriumsulfat getrocknet, filtriert und im Vakuum eingeengt. Die Titelverbindung wird mittels präparativer RP-HPLC (CromSil C18, Acetonitril/Wasser-Gradient) isoliert.

Ausbeute: 19 mg (17% d. Th.)

LC-MS: Rₜ = 3.06 min;

MS (ESI): m/z = 553 [M+H]⁺;

¹H-NMR (500 MHz, DMSO-d₆): δ = 12.59 (br. s, 1H), 8.99 (t, 1H), 7.60 (d, 1H), 7.56 (d, 2H), 7.29 (d, 2H), 7.20 (d, 1H), 5.55 (t, 1H), 4.89-4.80 (m, 1H), 4.20 (t, 1H), 3.99 (s, 2H), 3.85 (dd, 1H), 3.69 (t, 2H), 3.60 (t, 2H), 3.50 (t, 2H), 2.70 (t, 2H), 1.69-1.59 (m, 1H), 0.76 (d, 6H).

### B. Bewertung der pharmakoloeischen Wirksamkeit

Die erfindungsgemäßen verbindungen wirken insbesondere als selektive Inhibitoren des Blutgerinnungsfaktors Xa und hemmen nicht oder erst bei deutlich höheren Konzentrationen auch andere Serinproteasen wie Plasmin oder Trypsin.

Als "selektiv" werden solche Inhibitoren des Blutgerinnungsfaktors Xa bezeichnet, bei denen die IC₅₀-Werte für die Faktor Xa-Inhibierung gegenüber den IC₅₀-Werten für die Inhibierung anderer Serinproteasen, insbesondere Plasmin und Trypsin, um mindestens das 100-fache kleiner sind, wobei bezüglich der Testmethoden für die Selektivität Bezug genommen wird auf die im folgenden beschriebenen Testmethoden der Beispiele B.a.1) und B.a.2).

Die vorteilhaften pharmakologischen Eigenschaften der erfindungsgemäßen Verbindungen können durch folgende Methoden festgestellt werden:

### a) Testbeschreibungen (in vitro)

### a.1) Messung der Faktor Xa-Hemmung:

Die enzymatische Aktivität von humanem Faktor Xa (FXa) wird über die Umsetzung eines für den FXa-spezifischen chromogenen Substrats gemessen. Dabei spaltet der Faktor Xa aus dem chromogenen Substrat p-Nitroanilin ab. Die Bestimmungen werden wie folgt in Mikrotiterplatten durchgeführt:
Die Prüfsubstanzen werden in unterschiedlichen Konzentrationen in DMSO gelöst und für 10 Minuten mit humanem FXa (0.5 nmol/l gelöst in 50 mmol/l Tris-Puffer [*C*,*C*,*C*-Tris(hydroxymethyl)aminomethan], 150 mmol/l NaCl, 0.1% BSA [bovine serum albumine], pH = 8.3) bei 25°C inkubiert. Als Kontrolle dient reines DMSO. Anschließend wird das chromogene Substrat (150 µmol/l Pefachrome^{®} FXa der Firma Pentapharm) hinzugefügt. Nach 20 Minuten Inkubationsdauer bei 25°C wird die Extinktion bei 405 nm bestimmt. Die Extinktionen der Testansätze mit Prüfsubstanz werden mit den Kontrollansätzen ohne Prüfsubstanz verglichen und daraus die IC₅₀-Werte berechnet.

Wirkdaten aus diesem Test sind in der folgenden Tabelle 1 aufgeführt:

**Tabelle 1**

| **Beispiel Nr.** | **IC₅₀ [nM]** |
|---|---|
| 1 | 32 |
| 3 | 66 |
| 4 | 59 |

### a.2) Bestimmung der Selektivität:

Zum Nachweis der selektiven FXa-Inhibition werden die Prüfsubstanzen auf ihre Hemmung anderer humaner Serinproteasen wie Trypsin und Plasmin hin untersucht. Zur Bestimmung der enzymatischen Aktivität von Trypsin (500 mU/ml) und Plasmin (3.2 nmol/l) werden diese Enzyme in Tris-Puffer (100 mmol/l, 20 mmol/l CaCl₂, pH = 8.0) gelöst und für 10 Minuten mit Prüfsubstanz oder Lösungsmittel inkubiert. Anschließend wird durch Zugabe der entsprechenden spezifischen chromogenen Substrate (Chromozym Trypsin^{®} und Chromozym Plasmin^{®}; Fa. Roche Diagnostics) die enzymatische Reaktion gestartet und die Extinktion nach 20 Minuten bei 405 nm bestimmt. Alle Bestimmungen werden bei 37°C durchgeführt. Die Extinktionen der Testansätze mit Prüfsubstanz werden mit den Kontrollproben ohne Prüfsubstanz verglichen und daraus die IC₅₀-Werte berechnet.

### a.3) Bestimmung der antikoagulatorischen Wirkung:

Die antikoagulatorische Wirkung der Prüfsubstanzen wird *in vitro* in Human- und Kaninchenplasma bestimmt. Dazu wird Blut unter Verwendung einer 0.11 molaren Natriumcitrat-Lösung als Vorlage in einem Mischungsverhältnis Natriumcitrat/Blut 1:9 abgenommen. Das Blut wird unmittelbar nach der Abnahme gut gemischt und 10 Minuten bei ca. 2500 g zentrifugiert. Der Überstand wird abpipettiert. Die Prothrombinzeit (PT, Synonyme: Thromboplastinzeit, Quick-Test) wird in Gegenwart variierender Konzentrationen an Prüfsubstanz oder dem entsprechenden Lösungsmittel mit einem handelsüblichen Testkit (Hemoliance^{®} RecombiPlastin, Fa. Instrumentation Laboratory) bestimmt. Die Testverbindungen werden 3 Minuten bei 37°C mit dem Plasma inkubiert. Anschließend wird durch Zugabe von Thromboplastin die Gerinnung ausgelöst und der Zeitpunkt des Gerinnungseintritts bestimmt. Es wird die Konzentration an Prüfsubstanz ermittelt, die eine Verdoppelung der Prothrombinzeit bewirkt.

### b) Bestimmung der antithrombotischen Wirkung (in vivo)

### b.1) Arteriovenöses Shunt-Modell (Kaninchen):

Nüchterne Kaninchen (Stamm: Esd: NZW) werden durch intramuskuläre Gabe einer Rompun/Ketavet-Lösung narkotisiert (5 mg/kg bzw. 40 mg/kg). Die Thrombusbildung wird in einem arteriovenösen Shunt in Anlehnung an die von C.N. Berry et al. [Semin. Thromb. Hemost. 1996, 22, 233-241] beschriebene Methode ausgelöst. Dazu werden die linke Vena jugularis und die rechte Arteria carotis freipräpariert. Ein extracorporaler Shunt wird mittels eines 10 cm langen Venenkatheders zwischen den beiden Gefäßen gelegt. Dieser Katheder ist in der Mitte in einen weiteren, 4 cm langen Polyethytenschlauch (PE 160, Becton Dickenson), der zur Erzeugung einer thrombogenen Oberfläche einen aufgerauhten und zu einer Schlinge gelegten Nylonfaden enthält, eingebunden. Der extrakorporale Kreislauf wird 15 Minuten lang aufrechterhalten. Dann wird der Shunt entfernt und der Nylonfaden mit dem Thrombus sofort gewogen. Das Leergewicht des Nylonfadens ist vor Versuchsbeginn ermittelt worden. Die Prüfsubstanzen werden vor Anlegung des extrakorporalen Kreislaufs entweder intravenös über eine Ohrvene oder oral mittels Schlundsonde verabreicht.

### c) Bestimmung der Löslichkeit

### Benötigte Reagenzien:

- PBS-Puffer pH 7.4: 90.00 g NaCl p.a. (z.B. Fa. Merck, Art.-Nr. 1.06404.1000), 13.61 g KH₂PO₄ p.a. (z.B. Fa. Merck, Art.-Nr. 1.04873.1000) und 83.35 g 1 N NaOH (z.B. Fa. Bernd Kraft GmbH, Art.-Nr. 01030.4000) in einen 1 Liter-Messkolben einwiegen, mit Wasser auffüllen und ca. 1 Stunde rühren;
- Acetatpuffer pH 4.6: 5.4 g Natriumacetat x 3 H₂O p.a. (z.B. Fa. Merck, Art.-Nr. 1.06267.0500) in einen 100 ml-Messkolben einwiegen, in 50 ml Wasser lösen, mit 2.4 g Eisessig versetzen, auf 100 ml mit Wasser auffüllen, pH-Wert überprüfen und falls notwendig auf pH 4.6 einstellen;
- Dimethylsulfoxid (z.B. Fa. Baker, Art.-Nr. 7157.2500);
- destilliertes Wasser.

### Herstellung der Kalibrierlösungen:

*Herstellung der Ausgangslösung für Kalibrierlösungen (Stammlösung):* In ein 2 ml Eppendorf-Safe-Lock Tube (Fa. Eppendorf, Art.-Nr. 0030 120.094) werden ca. 0.5 mg der Testsubstanz genau eingewogen, zu einer Konzentration von 600 µg/ml mit DMSO versetzt (z.B. 0.5 mg Substanz + 833 µl DMSO) und bis zur vollständigen Lösung mittels eines Vortexers geschüttelt.

*Kalibrierlösung 1 (20 µglml):* 34.4 µl der Stammlösung werden mit 1000 µl DMSO versetzt und homogenisiert.

*Kalibrierlösung 2 (2.5 µglml):* 100 µl der Kalibrierlösung 1 werden mit 700 µl DMSO versetzt und homogenisiert.

### Herstellung der Probenlösungen:

*Probenlösung für Löslichkeit bis 10 g*/*l in PBS-Puffer pH 7.4:* In ein 2 ml Eppendorf-Safe-Lock Tube (Fa. Eppendorf, Art.-Nr. 0030 120.094) werden ca. 5 mg der Testsubstanz genau eingewogen und zu einer Konzentration von 5 g/l mit PBS-Puffer pH 7.4 versetzt (z.B. 5 mg Substanz + 500 µl PBS-Puffer pH 7.4).

*Probenlösung für Löslichkeit bis 10 g*/*l in Acetatpuffer pH 4.6:* In ein 2 ml Eppendorf-Safe-Lock Tube (Fa. Eppendorf, Art.-Nr. 0030 120.094) werden ca. 5 mg der Testsubstanz genau eingewogen und zu einer Konzentration von 5 g/l mit Acetatpuffer pH 4.6 versetzt (z.B. 5 mg Substanz + 500 µl Acetatpuffer pH 4.6).

*Probenlösung für Löslichkeit bis 10 g*/*l in Wasser:* In ein 2 ml Eppendorf-Safe-Lock Tube (Fa. Eppendorf, Art.-Nr. 0030 120.094) werden ca. 5 mg der Testsubstanz genau eingewogen und zu einer Konzentration von 5 g/l mit Wasser versetzt (z.B. 5 mg Substanz + 500 µl Wasser).

### Durchführung:

Die so hergestellten Probenlösungen werden 24 Stunden bei 1400 rpm mittels eines temperierbaren Schüttlers (z.B. Fa. Eppendorf Thermomixer comfort Art.-Nr. 5355 000.011 mit Wechselblock Art.-Nr. 5362.000.019) bei 20°C geschüttelt. Von diesen Lösungen werden jeweils 180 µl abgenommen und in Beckman Polyallomer Centrifuge Tubes (Art.-Nr. 343621) überführt. Diese Lösungen werden 1 Stunde mit ca. 223.000 x g zentrifugiert (z.B. Fa. Beckman Optima L-90K Ultracentrifuge mit Type 42.2 Ti Rotor bei 42.000 rpm). Von jeder Probenlösung werden 100 µl des Überstandes abgenommen und 1:5, 1:100 und 1:1000 mit dem jeweils verwendeten Lösungsmittel (Wasser, PBS-Puffer 7.4 oder Acetatpuffer pH 4.6) verdünnt. Es wird von jeder Verdünnung eine Abfüllung in ein geeignetes Gefäß für die HPLC-Analytik vorgenommen.

### Analytik:

Die Proben werden mittels RP-HPLC analysiert. Quantifiziert wird über eine Zwei-Punkt-Kalibrationskurve der Testverbindung in DMSO. Die Löslichkeit wird in mg/l ausgedrückt. Analysensequenz: 1) Kalibrierlösung 2.5 mg/ml; 2) Kalibrierlösung 20 µg/ml; 3) Probenlösung 1:5; 4) Probenlösung 1:100; 5) Probenlösung 1:1000. -

### HPLC-Methode für Säuren:

Agilent 1100 mit DAD (G1315A), quat. Pumpe (G 1311 A), Autosampler CTC HTS PAL, Degaser (G1322A) und Säulenthermostat (G1316A); Säule: Phenomenex Gemini C18, 50 mm x 2 mm, 5 µ; Temperatur: 40°C; Eluent A: Wasser/Phosphorsäure pH 2; Eluent B: Acetonitril; Flussrate: 0.7 ml/min; Gradient: 0-0.5 min 85% A, 15% B; Rampe: 0.5-3 min 10% A, 90% B; 3-3.5 min 10% A, 90% B; Rampe: 3.5-4 min 85% A, 15% B; 4-5 min 85% A, 15% B.

### HPLC-Methode für Basen:

Agilent 1100 mit DAD (G1315A), quat. Pumpe (G1311A), Autosampler CTC HTS PAL, Degaser (G1322A) und Säulenthermostat (G1316A); Säule: VDSoptilab Kromasil 100 C18, 60 mm x 2.1 mm, 3.5 µ; Temperatur: 30°C; Eluent A: Wasser + 5 ml Perchlorsäure/l; Eluent B: Acetonitril; Flussrate: 0.75 ml/min; Gradient: 0-0.5 min 98% A, 2% B; Rampe: 0.5-4.5 min 10% A, 90% B; 4.5-6 min 10% A, 90% B; Rampe: 6.5-6.7 min 98% A, 2% B; 6.7-7.5 min 98% A, 2% B.

### C. Ausführungsbeispiele für pharmazeutische Zusammensetzungen

Die erfindungsgemäßen Verbindungen können folgendermaßen in pharmazeutische Zubereitungen überführt werden:

### Tablette:

### Zusammensetzung:

100 mg der erfindungsgemäßen Verbindung, 50 mg Lactose (Monohydrat), 50 mg Maisstärke (nativ), 10 mg Polyvinylpyrrolidon (PVP 25) (Fa. BASF, Ludwigshafen, Deutschland) und 2 mg Magnesiumstearat.

Tablettengewicht 212 mg. Durchmesser 8 mm, Wölbungsradius 12 mm.

### Herstellung:

Die Mischung aus erfindungsgemäßer Verbindung, Lactose und Stärke wird mit einer 5%-igen Lösung (m/m) des PVPs in Wasser granuliert. Das Granulat wird nach dem Trocknen mit dem Magnesiumstearat 5 Minuten gemischt. Diese Mischung wird mit einer üblichen Tablettenpresse verpresst (Format der Tablette siehe oben). Als Richtwert für die Verpressung wird eine Presskraft von 15 kN verwendet.

### Oral applizierbare Suspension:

### Zusammensetzung:

1000 mg der erfindungsgemäßen Verbindung, 1000 mg Ethanol (96%), 400 mg Rhodigel^{®} (Xanthan gum der Firma FMC, Pennsylvania, USA) und 99 g Wasser.

Einer Einzeldosis von 100 mg der erfindungsgemäßen Verbindung entsprechen 10 ml orale Suspension.

### Herstellung:

Das Rhodigel wird in Ethanol suspendiert, die erfindungsgemäße Verbindung wird der Suspension zugefügt. Unter Rühren erfolgt die Zugabe des Wassers. Bis zum Abschluß der Quellung des Rhodigels wird ca. 6 h gerührt.

### Oral applizierbare Lösung:

### Zusammensetzung:

500 mg der erfindungsgemäßen Verbindung, 2.5 g Polysorbat und 97 g Polyethylenglycol 400. Einer Einzeldosis von 100 mg der erfindungsgemäßen Verbindung entsprechen 20 g orale Lösung.

### Herstellung:

Die erfindungsgemäße Verbindung wird in der Mischung aus Polyethylenglycol und Polysorbat unter Rühren suspendiert. Der Rührvorgang wird bis zur vollständigen Auflösung der erfindungsgemäßen Verbindung fortgesetzt.

### i.v.-Lösung:

Die erfindungsgemäße Verbindung wird in einer Konzentration unterhalb der Sättigungslöslichkeit in einem physiologisch verträglichen Lösungsmittel (z.B. isotonische Kochsalzlösung, Glucoselösung 5% und/oder PEG 400-Lösung 30%) gelöst. Die Lösung wird steril filtriert und in sterile und pyrogenfreie Injektionsbehältnisse abgefüllt.

## Patentansprüche

1. Verbindung der Formel (I) 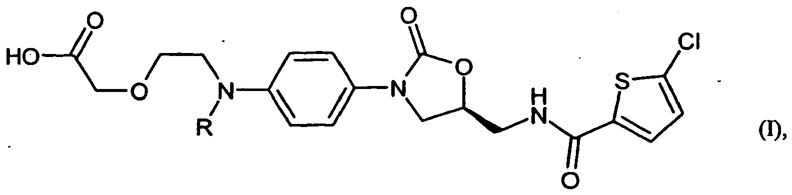 in welcher
R für Wasserstoff, (C₁-C₆)-Alkyl, (C₁-C₆)-Alkanoyl, (C₁-C₆)-Alkoxycarbonyl, Mono-oder Di-C₁-C₆)-alkylaminocarbonyl, Benzoyl oder Heteroaroyl steht, wobei Benzoyl und Heteroaroyl ihrerseits mit Halogen, Cyano, (C₁-C₄)-Alkyl oder (C₁-C₄)-Alkoxy substituiert sein können,
sowie ihre Salze, Solvate und Solvate der Salze.

2. Verbindung der Formel (I) nach Anspruch 1, in welcher
R für Wasserstoff, Isobutylaminocarbonyl oder 5-Chlor-2-thienylcarbonyl steht,
sowie ihre Salze, Solvate und Solvate der Salze.

3. Verbindung der Formel (I) nach Anspruch 1 mit folgender Struktur: 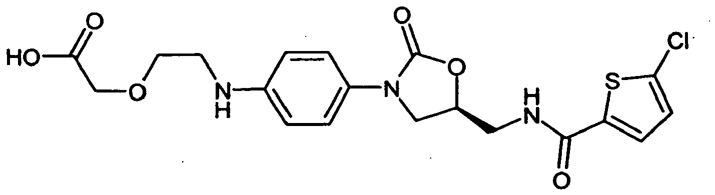 sowie ihre Salze, Solvate und Solvate der Salze.

4. Verfahren zur Herstellung von Verbindungen der Formel (I), wie in Anspruch 1 definiert, **dadurch gekennzeichnet, dass** man die Verbindung der Formel (II) 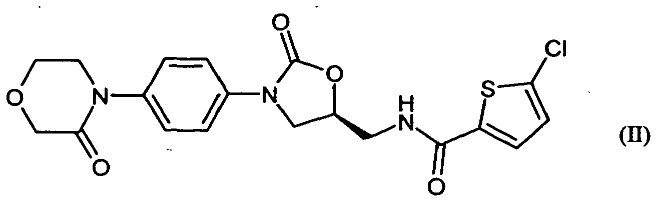 durch Hydrolyse in die Verbindung der Formel (I-A) 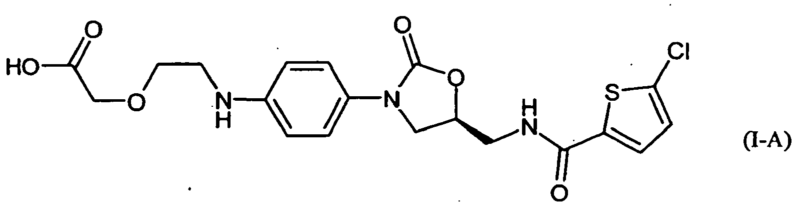 überführt und diese dann in einem inerten Lösungsmittel, gegebenenfalls in Gegenwart einer Base, mit einer Verbindung der Formel (III) 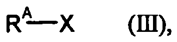 in welcher
R^{A} für (C₁-C₆)-Alkyl, (C₁-C₆)-Alkanoyl, (C₁-C₆)-Alkoxycarbonyl, Di-(C₁-C₆)-alkyl-aminocarbonyl, Benzoyl oder Heteroaroyl steht, wobei Benzoyl und Heteroaroyl ihrerseits mit Halogen, Cyano, (C₁-C₄)-Alkyl oder (C₁-C₄)-Alkoxy substituiert sein können,
und X für eine Abgangsgruppe wie beispielsweise Halogen steht,
oder im Falle, dass R für Mono-(C₁-C₆)-alkylaminocarbonyl steht, mit einer Verbindung der Formel (IV) 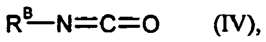 in welcher
R^{B} für (C₁-C₆)-Alkyl steht,
umsetzt
und die resultierenden Verbindungen der Formel (I) bzw. (I-A) gegebenenfalls mit den entsprechenden (i) Lösungsmitteln und/oder (ii) Basen oder Säuren in ihre Solvate, Salze und/oder Solvate der Salze überführt.

5. Verbindung der Formel (I), wie in Anspruch 1 definiert, zur Behandlung und/oder Prophylaxe von Krankheiten.

6. Verwendung einer Verbindung der Formel (I), wie in Anspruch 1 definiert, zur Herstellung eines Arzneimittels zur Behandlung und/oder Prophylaxe von thromboembolischen Erkrankungen.

7. Verwendung einer Verbindung der Formel (I), wie in Anspruch 1 definiert, zur Verhinderung der Blutkoagulation in vitro.

8. Arzneimittel enthaltend eine Verbindung der Formel (I), wie in Anspruch 1 definiert, in Kombination mit einem inerten, nicht-toxischen, pharmazeutisch geeigneten Hilfsstoff.

9. Arzneimittel enthaltend eine Verbindung der Formel (I), wie in Anspruch 1 definiert, in Kombination mit einem weiteren Wirkstoff.

10. Arzneimittel nach Anspruch 8 oder 9 zur Behandlung und/oder Prophylaxe von thromboembolischen Erkrankungen.

## Claims

1. Compound of the formula (I) 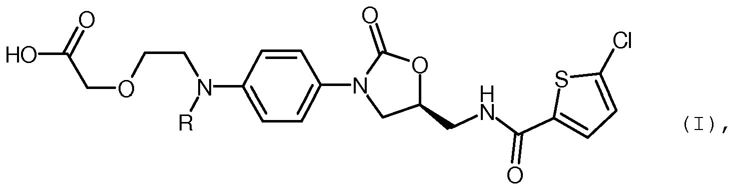 in which
R represents hydrogen, (C₁-C₆)-alkyl, (C₁-C₆)-alkanoyl, (C₁-C₆)-alkoxycarbonyl, mono- or di-(C₁-C₆)-alkylaminocarbonyl, benzoyl or heteroaroyl, where benzoyl and heteroaroyl for their part may be substituted by halogen, cyano, (C₁-C₄)-alkyl or (C₁-C₄) -alkoxy,
and salts, solvates and solvates of the salts thereof.

2. Compound of the formula (I) according to Claim 1 in which
R is hydrogen, isobutylaminocarbonyl or 5-chloro-2-thienylcarbonyl,
and salts, solvates and solvates of the salts thereof.

3. Compound of the formula (I) according to Claim 1 having the structure below: 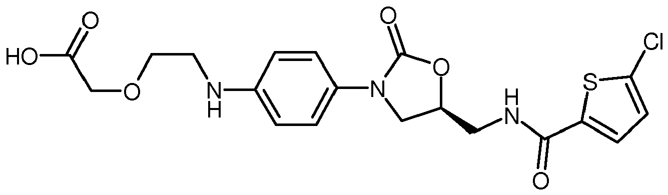 and salts, solvates and solvates of the salts thereof.

4. Process for preparing compounds of the formula (I), as defined in Claim 1, **characterized in that** the compound of the formula (II) 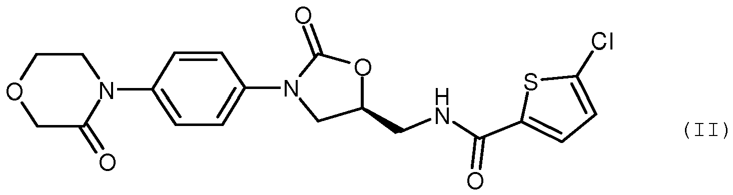 is converted by hydrolysis into the compound of the formula (I-A) 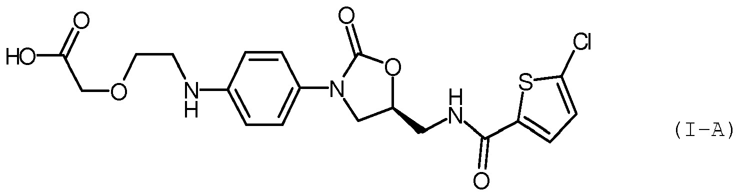 and this is then, in an inert solvent, if appropriate in the presence of a base, reacted with a compound of the formula (III) 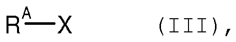 in which
R^{A} represents (C₁-C₆)-alkyl, (C₁-C₆) -alkanoyl, (C₁-C₆)-alkoxycarbonyl, di-(C₁-C₆)-alkylaminocarbonyl, benzoyl or heteroaroyl, where benzoyl and heteroaroyl for their part may be substituted by halogen, cyano, (C₁-C₄)-alkyl or (C₁-C₄)-alkoxy,
and X represents a leaving group, such as, for example, halogen,
or, if R represents mono-(C₁-C₆)-alkylaminocarbonyl, with a compound of the formula (IV) 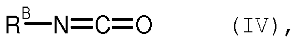 in which
R^{B} represents (C₁-C₆)-alkyl,
and the resulting compounds of the formula (I) or (I-A) are, if appropriate, converted with the appropriate (i) solvents and/or (ii) bases or acids into their solvates, salts and/or solvates of the salts.

5. Compound of the formula (I) as defined in Claim 1 for the treatment and/or prophylaxis of diseases.

6. Use of a compound of the formula (I) as defined in Claim 1 for preparing a medicament for the treatment and/or prophylaxis of thromboembolic disorders.

7. Use of a compound of the formula (I) as defined in Claim 1 for preventing blood coagulation in vitro.

8. Medicament comprising a compound of the formula (I) as defined in Claim 1 in combination with an inert nontoxic, pharmaceutically suitable auxiliary.

9. Medicament comprising a compound of the formula (I) as defined in Claim 1 in combination with a further active compound.

10. Medicament according to Claim 8 or 9 for the treatment and/or prophylaxis of thromboembolic disorders.

## Revendications

1. Composé de formule (I) 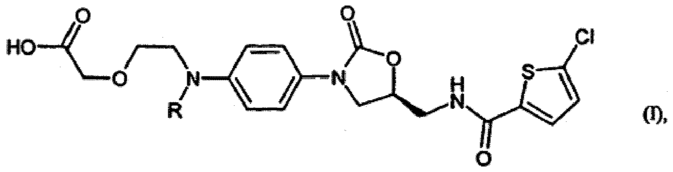 dans laquelle
R représente hydrogène, (C₁-C₆)-alkyle, (C₁-C₆)-alcanoyle, (C₁-C₆)-alcoxycarbonyle, mono-(C₁-C₆)-alkylaminocarbonyle ou di-(C₁-C₆)-alkylaminocarbonyle, benzoyle ou hétéroaroyle, benzoyle et hétéroaroyle pouvant à leur tour être substitués par halogène, cyano, (C₁-C₄)-alkyle ou (C₁-C₄)-alcoxy,
ainsi que ses sels, solvates et les solvates des sels.

2. Composé de formule (I) selon la revendication 1, dans laquelle
R représente hydrogène, isobutylaminocarbonyle ou 5-chloro-2-thiénylcarbonyle,
ainsi que ses sels, solvates et les solvates des sels.

3. Composé de formule (I) selon la revendication 1 présentant la structure suivante 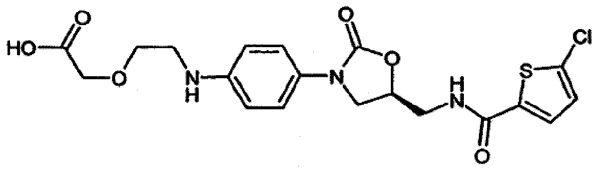 ainsi que ses sels, solvates et les solvates des sels.

4. Procédé pour la préparation de composés de formule (I) tels que définis dans la revendication 1, **caractérisé en ce qu'**on transforme le composé de formule (II) 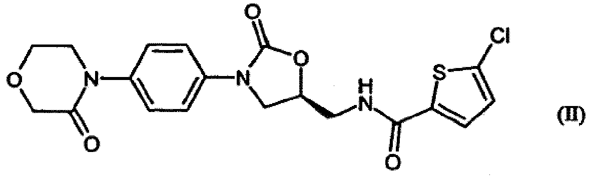 par hydrolyse en composé de formule (I-A) 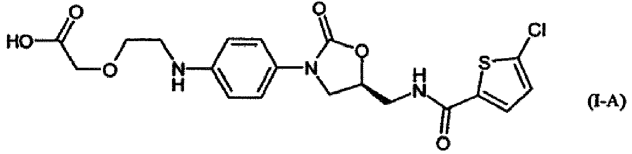 puis on transforme celui-ci dans un solvant inerte, le cas échéant en présence d'une base, avec un composé de formule (III)
R^{A}-X (III),
dans laquelle
R^{A} représente (C₁-C₆)-alkyle, (C₁-C₆)-alcanoyle, (C₁-C₆) -alcoxycarbonyle, di-(C₁-C₆)-alkylaminocarbonyle, benzoyle ou hétéroaroyle, benzoyle et hétéroaroyle pouvant à leur tour être substitués par halogène, cyano, (C₁-C₄)-alkyle ou (C₁-C₄)-alcoxy et
X représente un groupe partant tel que par exemple halogène,
ou dans le cas où R représente mono-(C₁-C₆)-alkylaminocarbonyle, avec un composé de formule (IV)
R^{B}-N=C=O (IV),
dans laquelle
R^{B} représente (C₁-C₆)-alkyle,
et on transforme les composés résultants de formule (I) ou, selon le cas, (I-A) le cas échéant avec (i) les solvants et/ou (ii) les bases ou acides correspondants en leurs solvates, sels et/ou solvates des sels.

5. Composé de formule (I), tel que défini dans la revendication 1, destiné au traitement et/ou à la prophylaxie de maladies.

6. Utilisation d'un composé de formule (I), tel que défini dans la revendication 1, pour la préparation d'un médicament destiné au traitement et/ou à la prophylaxie de maladies thromboemboliques.

7. Utilisation d'un composé de formule (I), tel que défini dans la revendication 1, destinée à empêcher la coagulation du sang in vitro.

8. Médicament contenant un composé de formule (I), tel que défini dans la revendication 1, en combinaison avec un adjuvant inerte, non toxique, pharmaceutiquement approprié.

9. Médicament contenant un composé de formule (I), tel que défini dans la revendication 1, en combinaison avec une autre substance active.

10. Médicament selon la revendication 8 ou 9 destiné au traitement et/ou à la prophylaxie de maladies thromboemboliques.
